# EUROPEAN PATENT APPLICATION

(11) **EP 4 379 355 A1**
(43) Date of publication of application: **05.06.2024**
(21) Application number: 22210255.0
(22) Date of filing: 29.11.2022
(51) Int. Cl.: G01N 21/64, C12Q 1/68

(54) **HIGH THROUGHPUT ANALYSIS OF SINGLE MOLECULE EVENTS**

(71) Applicant: Gnothis Holding AG, 6330 Cham (CH)
(72) Inventor: The designation of the inventor has not yet been filed
(74) Representative: Weickmann & Weickmann PartmbB

(57) **Abstract**

The present disclosure relates to the analysis of molecular events as observed by irradiating a sample (18) with an excitation radiation (16) and detecting emission radiation (22) from the sample induced by said excitation radiation, wherein the excitation radiation pathway from said radiation source to said plurality of samples and the emission radiation pathway from said plurality of samples to said detection means (26) do not overlap.

## Description

The present disclosure relates to the analysis of molecular events as observed by irradiating a sample with an excitation radiation and detecting emission radiation from the sample induced by said excitation radiation, wherein the excitation radiation pathway from said radiation source to said plurality of samples and the emission radiation pathway from said plurality of samples to said detection means do not overlap.

### Background

Sequencing of the human genome or the genome of other organisms and the determination and comparison of individual sequence variants requires the provision of sequencing methods which firstly are fast and secondly can be employed routinely and cost-effectively.

The high demand for cost-efficient sequencing has driven the development of high-throughput sequencing technologies that parallelize the sequencing process producing a plurality of sequences concurrently. Examples of these sequencing technologies are massively parallel signature sequencing (Lynx Therapeutics), polony sequencing (Life Technologies), 454 pyrosequencing (Roche Diagnostics), illumina sequencing (Solexa Inc.), sequencing by ligation (Life Technologies), ion torrent semiconductor sequencing (Life Technologies) or DNA nanoball sequencing (Complete Genomics). These technologies allow rapid analysis of a consensus sequence in a nucleic acid population. Mutations existing in minority sequences in the nucleic acid population to be analysed, e.g., in a minority of cellular genomes, however, will not be detected since they are obscured by the majority of other sequences present in the population.

In order to address this issue, single molecule sequencing processes in several different formats have been developed. Several of these processes are within the field of fluorescence spectroscopy (FCS) and involve detection and analysis of single molecules by fluorescence. Typically, nucleic acid-polymerizing enzymes and/or nucleic acid-degrading enzymes and fluorescence-labelled nucleic acids and/or nucleotide building blocks are used for individually determining the sequence of a single nucleic acid molecule on the basis of a time-dependent change in fluorescence when nucleotide building blocks are incorporated into or cleaved off from a nucleic acid molecule. Single molecule sequencing processes and devices adapted for performing such processes are e.g., described in co-owned applications WO 2002/097406, WO 2003/052137, WO 2006/013110, WO 2013/131888, WO 2015/104245, WO 2017/001407, and WO 2018/104301.

US 7,259,847 B2, the disclosure of which is incorporated by reference, discloses a method for determining luminescent molecules by optical excitation in confocal measurement volumes comprising the steps of
(a) providing a sample comprising luminescent molecules,
(b) irradiating the sample with an optical excitation device comprising a light source, a diffractive optical element for splitting light passing through into multiple foci and a focusing optical arrangement for focusing multiple light beams passing through into multiple confocal volume elements, and ,
(c) capturing emission radiation from the multiple confocal volume elements.

There is no disclosure of an embodiment, wherein the excitation radiation pathway and the emission radiation pathway do not overlap.

It was one of several objects of the present disclosure to provide methods and devices for the high-throughput analysis of single molecule events.

### Summary of the Invention

In a first aspect, the present disclosure relates to a method for analyzing a single molecule event comprising:
(a) providing a plurality of samples where a single molecule event takes place,
(b) irradiating said plurality of samples with an optical excitation device comprising a radiation source, a diffractive optical element for splitting excitation radiation into multiple individual radiation beams and an optical arrangement for directing said multiple individual radiation beams to said plurality of samples,
(c) capturing emission radiation from said plurality of samples by a detection means, and
(d) analyzing the captured emission radiation,
wherein the excitation radiation pathway from said radiation source to said plurality of samples and the emission radiation pathway from said plurality of samples to said detection means do not overlap.

In a particular embodiment, the single molecule event comprises a sequence analysis of a single nucleic acid molecule.

A further aspect relates to a device for analyzing a single molecule event comprising:
- means for providing a plurality of samples adapted for a single molecule event,
- an optical excitation device comprising a radiation source adapted for emitting excitation radiation, a diffractive optical element for splitting said excitation radiation into multiple individual radiation beams and an optical arrangement for directing, e.g., focusing, said multiple individual radiation beams to said plurality of samples,
- a detection means adapted for capturing emission radiation from said plurality of samples, and
- an analyzing means adapted for analyzing the captured emission radiation,
wherein the excitation radiation pathway from said radiation source to said plurality of samples and the emission radiation pathway from said plurality of samples to said detection means do not overlap.

In a particular embodiment, the device is adapted for the sequence analysis of a single nucleic acid molecule.

A further aspect relates to the use of the above method or the above device for providing a high-throughput analysis of a single molecule event.

### Items of the Specification

1. A method for analyzing a single molecule event comprising:
   (a) providing a plurality of samples where a single molecule event takes place,
   (b) irradiating said plurality of samples with an optical excitation device comprising a radiation source, a diffractive optical element for splitting excitation radiation into multiple individual radiation beams and an optical arrangement for directing said multiple individual radiation beams to said plurality of samples,
   (c) capturing emission radiation from said plurality of samples by a detection means, and
   (d) analyzing the captured emission radiation,
   wherein the excitation radiation pathway from said radiation source to said plurality of samples and the emission radiation pathway from said plurality of samples to said detection means do not overlap.
2. The method of item 1, wherein the excitation radiation pathway from said radiation source originates from a plane above the emission pathway from said plurality of samples to said detection means.
3. The method of item 1 or 2, wherein said plurality of samples is present on a support.
4. The method of item 3, wherein said support comprises a substrate and a plurality of sample spots on the surface of the support, wherein the sample spots are spatially separated from another and wherein said samples are present on said sample spots.
5. The method of item 4, wherein said substrate is optically transparent.
6. The method of item 4 or 5, wherein a component of said single molecule event is immobilized to said at least one sample spot.
7. The method of item 6, wherein said immobilized component comprises a single biological moiety, e.g., a single biomolecule.
8. The method of any one of the preceding items, wherein said radiation source comprises at least one laser.
9. The method of any one of the preceding items, wherein said plurality of samples comprises at least 10, at least 100, at least 1,000, at least 10⁴, at least 10⁵ or at least 10⁶ and up to 10⁸, 10⁹, up to 10¹⁰ or up to 10¹¹ individual samples.
10. The method of any one of the preceding items, wherein an individual radiation beam of the excitation radiation is split into about 10 to about 1000, or about 50 to about 500, e.g., about 100 individual radiation beams.
11. The method of any one of the preceding items, wherein an individual radiation beam of the excitation radiation is directed to an individual sample or to a group comprising multiple individual samples.
12. The method of any one of the preceding items, wherein an individual radiation beam of the excitation radiation is directed to about 10 to about 10⁶, or about 100 to about 10⁵ groups comprising multiple individual samples.
13. The method of item 11 or 12, wherein a group comprises up to about 10⁶ individual samples, and particularly about 1,000 to about 10⁶, or about 5,000 to about 10⁵, e.g., about 10,000 individual samples.
14. The method of any one of the preceding items, wherein step (a) comprises focusing said multiple individual radiation beams to said plurality of samples.
15. The method of any one of the preceding items, wherein said multiple beams generated by the optical diffractive element are passed to said plurality of samples through an optically transparent substrate of a support on which the plurality of samples is located.
16. The method of item 15, wherein the plurality of samples is located on the surface of the support that is distal to the surface of the support through which the excitation radiation enters the support.
17. The method of item 16, wherein an individual radiation beam of the excitation radiation generates an individual evanescent field on the surface of the support on which the plurality of samples is located.
18. The method of item 17, wherein said individual evanescent field has a diameter of about 1 nm to about 100 µm or about 10 nm to about 10 µm.
19. The method of item 17 or 18, wherein said individual evanescent field covers up to about 10⁶ individual samples, and particularly about 1,000 to about 10⁶, or about 5,000 to about 20,000, e.g., about 10,000 individual samples.
20. The method of any one of items 17-19, wherein said individual evanescent field is generated via total internal reflection.
21. The method of any one of items 17-20, wherein a plurality of individual evanescent fields is generated.
22. The method of item 21, wherein the individual evanescent fields do not substantially overlap.
23. The method of item 21, wherein the overlap between two adjacent fields is about 20% (by area) or less, or about 10% (by area) or less.
24. The method of any one of the preceding items, wherein said diffractive optical element is an optical grating.
25. The method of any one of the preceding items, wherein said detection means comprises a detector matrix comprising a plurality of detection pixels.
26. The method of item 25, wherein said detection pixels are optically projected on the sample spots on the surface of the support.
27. The method of any one of the preceding items, wherein the detection means provides optical foci on the individual sample spots.
28. The method of any one of the preceding items, wherein at least one reaction space is provided around said plurality of samples, wherein said reaction space comprises a medium and components for said single molecule event.
29. The method of item 28, wherein said reaction space is a flow cell.
30. The method of item 28 or 29, wherein a single reaction space is provided around said plurality of samples.
31. The method of any one of items 28-30, wherein said reaction space has a depth of about 10 nm to about 1,000 µm.
32. The method of any one of items 28-31, wherein said emission radiation pathway passes through said reaction space.
33. The method of any one of the preceding items, wherein said single molecule event comprises a sequence analysis of a single nucleic acid molecule.
34. A device for analyzing a single molecule event comprising:
   - means for providing a plurality of samples adapted for a single molecule event,
   - an optical excitation device comprising a radiation source adapted for emitting excitation radiation, a diffractive optical element for splitting said excitation radiation into multiple individual radiation beams and an optical arrangement for directing said multiple individual radiation beams to said plurality of samples,
   - a detection means adapted for capturing emission radiation from said plurality of samples, and
   - an analyzing means adapted for analyzing the captured emission radiation, wherein the excitation radiation pathway from said radiation source to said plurality of samples and the emission radiation pathway from said plurality of samples to said detection means do not overlap.
35. The device of item 34 adapted for performing the method of any one of items 1-33.
36. The device of item 34 or 35 adapted for performing a single molecule nucleic acid sequence analysis.
37. Use of the method of any one of items 1-33 or the device of any one of items 34-36 for providing a high-throughput analysis of a single molecule event.
38. The use of item 37, wherein the high-throughput analysis comprises a parallel analysis of about 10⁶ to about 10¹¹ individual samples.
39. The use of item 38, wherein the high-throughput analysis comprises a parallel analysis of about 10⁶ to about 10¹¹ individual sample spots on a single support.
40. The use of item 38 or 39, wherein the high-throughput analysis is carried out in a single reaction space.

### Detailed Description

The present disclosure provides methods and devices for analyzing a single molecule event, wherein the single molecule event is associated with the emission of an electromagnetic radiation from a sample, e.g., a sample spot on a support comprising the components of the single molecule event. In particular embodiments, the single molecule event encompasses a reaction of a biological moiety which is associated with emission of a characteristic electromagnetic radiation.

The present disclosure addresses the need of providing improved high-throughput methods in the analysis of a single molecule event as observed by irradiating a sample with excitation radiation, and simultaneously detecting the resulting emission radiation from the sample as induced by said irradiation. The concept described in this disclosure includes the parallel analysis of a plurality of groups of individual sample spots on a single support, which each group can have up to 10⁶ sample spots and wherein each group may be illuminated by a single evanescent field. The diffractive optical element splitting the excitation radiation beam may create many groups, e.g., up to 10⁴, up to 10⁵ or even more. Hence, the total number of sample spots to be analyzed according to the present disclosure can be about 10⁶ up to about 10¹¹, particularly on a single support. Further, the present disclosure provides a reaction space comprising a sample solution, e.g., a liquid medium in contact with the sample spots that requires only a smaller volume allowing a higher flexibility with regards to the sample solution distribution.

In particular, the present disclosure provides a device and process for irradiating a plurality of samples with excitation radiation that is generated by a radiation source, e.g., a radiation source comprising at least one laser, split into multiple individual radiation beams by an optical diffractive element, wherein the multiple individual radiation beams are directed to said plurality of samples. The samples comprise components capable of emitting emission radiation that is captured by a detection means and analyzed.

According to the present disclosure, the excitation radiation pathway from the radiation source to the plurality of samples and the emission radiation pathway from the plurality of samples to the detection means do not overlap. Thereby, it is possible to achieve a much larger surface area upon which the sample spots are situated, and hence, the number of samples analyzed is greatly increased.

In certain embodiments, the excitation radiation pathway from the radiation source originates from a plane above, i.e., spatially above the emission pathway from the plurality of samples to the detection means.

In certain embodiments, the plurality of samples comprises at least 10, at least 100, at least 1,000, at least 10⁴ , at least 10⁵ or at least 10⁶ and up to 10⁸, 10⁹, up to 10¹⁰ or up to 10¹¹ individual samples.

The samples are typically present on a support. The support may comprise a substrate and a plurality of sample spots on the support surface that are spatially separated from another. In certain embodiments, the support surface is formed by the substrate and the sample spots and wherein said samples are present on said sample spots. In certain embodiments, the substrate forms a contiguous area in which the sample spots are distributed. An individual sample spot on the support surface is surrounded by the substrate, which differs from the sample spot, e.g., in respect of the material and/or the surface. Typically, the substrate is adapted for inhibiting and/or blocking adhesion of biomolecules such as polypeptides whereas the sample spots are adapted for allowing adhesion of desired biomolecules.

On a sample spot, a component taking part in the single molecule event, e.g., a biological moiety is immobilized. In certain embodiments, the component comprises a biomolecule, particularly a single biomolecule. The term "single biomolecule" encompasses a single molecular entity such as a polypeptide or a complex consisting of a plurality of individual units, e.g., individual molecular entities wherein the individual units form together a functional biological moiety.

In certain embodiments, the support is a substantially planar support, i.e., it does not comprise elevations or recesses of about 1000 nm or more or of about 100 nm or more. In further embodiments, the support is a structured support, e.g., a support comprising recesses such as wells that may have a volume of about 5 × 10⁻²⁴ liters to about 1 × 10⁻¹⁵ liter, or pillars of height of about 1 nm to 500 nm. In principle, the support may have any design, as long as a reaction space can be formed which enables the occurrence of a single molecule event on said at least two sample spots where the single biomolecules is immobilized.

In certain embodiments, the substrate is an optically transparent material, i.e., a material that is substantially transparent for electromagnetic radiation, e.g., radiation in the visible range and/or radiation in the near-infrared range. In certain embodiments, the substrate comprises a material having an absolute refractive index of at least 1.01, e.g., about 1.5 to about 3 in the visible range or about 1.5 to about 4 in the near-infrared range. In further embodiments, the substrate is an optically opaque material, e.g., a metal or semi-metal such as silicon.

In particular embodiments, the substrate comprises a non-electrically conductive material. Specific examples are glass, quartz, plastic, metal oxide-based materials, e.g., silicon dioxide-based materials such as glass, silica, or quartz, or composites comprising said materials. In further embodiments, the substrate comprises an electrically conductive material, e.g., an optically transparent material such as indium tin oxide.

Typically, the substrate has a thickness of about 10 µm to about 5 mm, particularly about 20 µm to about 2 mm.

The surface of the support comprises a plurality of sample spots that are spatially separated from another by the substrate surface. The sample spots are adapted for attachment of biomolecules. In certain embodiments, the support comprises a plurality of sample spots, e.g., at least 10, at least 100, at least 1,000, at least 10⁴, at least 10⁵ or at least 10⁶ and up to 10⁸, 10⁹, up to 10¹⁰ or up to 10¹¹ individual sample spots.

In certain embodiments, a sample spot comprises or consists of at least one electrically conductive material, e.g., a metal including a single metal or a combination of metals, e.g., an alloy or mixture of a plurality of different metals. For example, a metal that is capable of attachment to a sulfur containing moiety, e.g., in the form of a thiol or a disulfide, or a metal that is capable of attachment to a chelating moiety, e.g., a poly-histidine tag, is suitable. In certain embodiments, the metal has a positive electrochemical potential. Specific examples of suitable metals include but are not limited to Au, Cu, Ni, Pt, Pd, Rh, Ir, Os, Ru, and any combination thereof comprising at least two of said metals.

In certain embodiments, a sample spot comprises or consists of at least one at least one metal oxide including a single metal oxide or a combination of metal oxides. For example, a metal oxide that is capable of attachment to a phosphorus containing moiety, e.g., in the form of a phosphonic acid or phosphonic acid ester, or a metal oxide that is capable of attachment to a chelating moiety, e.g., a poly-histidine tag, is suitable. Specific examples of suitable metal oxides include TiO₂ and NiO.

In further embodiments, a sample spot comprises or consists of at least one non-electrically conductive material.

Sample spots may be prepared by vapour deposition of metals, which are vaporized on the support covered by a grid mask, which may be produced by electron beam lithography or equivalent technologies. The size of holes in the grid mask may correspond to the size of the spots on the support surface. Alternatively, the spots on the support may be prepared by site-specific deposition of nanoparticles, e.g., having a size of 2-10 nm, by precision pipetting of particles on the support, particularly on a support having a planar surface.

In particular embodiments, a sample spot has a size that is suitable for the attachment of a single biomolecule. In those embodiments, a sample spot has a diameter of about 1 nm to about 30 nm, particularly a diameter of about 2 nm to about 20 nm.

In certain embodiments, the sample spot is a separate object on the surface of the substrate. In certain embodiments, the sample spot has a lower face proximal to the substrate and an upper face distal to the substrate, wherein the distance between the lower and upper face defines the height of the sample spot. In particular embodiments, the height is about 50 pm to about 500 nm, particularly about 100 pm to about 20 nm, and more particularly about 500 pm to about 10 nm, e.g., about 2 nm.

The sample spots on the support may be adapted for the attachment of a biomolecule, e.g., by a covalent or non-covalent attachment. The biomolecule may be selected from polypeptides, nucleic acids, carbohydrates, and any combination thereof, e.g., a glycosylated polypeptide, or a ribonucleoprotein. In certain embodiments, the biomolecule is a complex consisting of several individual units, e.g., several polypeptide units, or several polypeptide and nucleic acid units.

In specific embodiments, the biomolecule is a nucleic acid-polymerizing enzyme, particularly a DNA polymerase or an RNA polymerase, or a nucleic acid-polymerizing molecule complex, particularly a DNA- or RNA-polymerizing complex comprising a nucleic acid-polymerizing enzyme and a nucleic acid molecule. In particular embodiments, the biomolecule is a DNA polymerase having a DNA binding cleft, particularly a Family A DNA polymerase including, but not limited to a Klenow, Taq or T7 DNA polymerase or any genetically modified version thereof, or a Family B polymerase including, but not limited to a therminator, Phi29, RB-69 or T4 DNA polymerase or any genetically modified version thereof. Reference is made in this context to US 7,745,116 B2, the content of which is herein incorporated by reference.

In further embodiments, the biomolecule is a nucleic acid-degrading enzyme, particularly an exonuclease, or a nucleic acid-degrading molecule complex, particularly a DNA- or RNA-degrading molecule complex comprising a nucleic acid-degrading enzyme and a nucleic acid molecule.

In still further embodiments, the biomolecule is a gene editing enzyme, particularly a Cas nuclease such as a Cas3, Cas9, Cas10, or Cas12 nuclease or any genetically modified version thereof, e.g., a Cas nickase, or a gene editing complex comprising a gene editing enzyme and nucleic acid molecule, e.g., a guide RNA and/or a target nucleic acid.

The single molecule event to be detected takes place in a sample that comprises the required components of the event, for example, a biomolecule and optionally small molecules. At least one of the components is a luminescent component that comprises a luminescent group, i.e., a group that is capable of emitting radiation in response of being irradiated by the excitation radiation. In certain embodiments, the luminescent component is a compound carrying a luminescent labeling group. In certain embodiments, the luminescent component is a compound that is capable of luminescence per se.

In certain embodiments, the luminescent component of the single molecule event may be a reactant, reaction intermediate and/or reaction product. In particular embodiments, the luminescent component is a fluorescent component, i.e., a component that is capable of emitting fluorescence radiation in response of being irradiated by the excitation radiation. In certain embodiments, the fluorescent component is a compound carrying a fluorescent labeling group. In certain embodiments, the fluorescent component is a compound that is capable of fluorescence per se.

In particular embodiments, the sample of the single molecule event comprises a plurality of different luminescent components, wherein at least some of said luminescent components have partially overlapping spectra of emission radiation. For example, the sample may comprise a plurality of different fluorescent components wherein at least some of said fluorescent components have distinguishable and partially overlapping fluorescence emission spectra.

The at least one luminescent component may be present in the sample in immobilized form and/or free form. In certain embodiments, the least one luminescent component is present in free form.

According to the present disclosure, the excitation radiation is split into multiple individual radiation beams by an optical diffractive element. In certain embodiments, the excitation radiation is split into about 10 to about 1000, about 50 to about 500 and e.g., about 100 individual radiation beams.

In certain embodiments, the optical diffractive element is an optical grating, e.g., a three-dimensional optical grating optionally applied to an optically transparent carrier. Light passing through the optical diffractive element is split whereby a predetermined diffraction pattern of multiple radiation beams is generated, that may form a desired arrangement of multiple optical foci in an object plane by means of constructive and destructive interference. The production of suitable diffractive optical elements is described, for example in the Dissertation by F. Nikolaef at the Chalmers Institute of Technologies (1999), in the Dissertation by M. Johansson at the Chalmers Institute of Technologies (2001) and in the publication Johansson and Hard (Applied Optics 38(1999), 1302-1310). Suitable materials for producing the optical elements are plastics, glass and composites or other materials having optical transparency for a given wavelength which can be processed by means of photolithographic etching.

An individual radiation beam of the excitation radiation may be directed to an individual sample or to a group comprising multiple individual samples. In certain embodiments, a group of samples comprises up to about 10⁶ individual samples, and particularly about 1,000 to about 10⁶, or about 5,000 to about 10⁵, e.g., about 10,000 individual samples.

In certain embodiments, step (a) of the above method comprises focusing the multiple individual radiation beams generated by diffractive optical element to the plurality of samples. In further embodiments, step (a) comprises directing the multiple individual radiation beams generated by diffractive optical element to the plurality of samples without focusing, e.g., by using one or more highly spatially coherent laser for generating the excitation radiation.

In certain embodiments, the multiple beams of the excitation radiation generated by the optical diffractive element are passed to the samples passes through an optically transparent substrate of a support on which the samples are located. In particular embodiments, the samples are located on a surface of the support that is distal to the surface of the support through which the multiple beams of the excitation radiation enter the support. In those embodiments, an individual radiation beam of the excitation radiation generates an individual evanescent field on the surface of the support on which the samples are located. An individual evanescent field may be generated via total internal reflection In certain embodiments, an individual evanescent field may have a diameter of about 1 nm to about 100 µm or about 10 nm to about 10 µm. In particular embodiments, an individual evanescent field may cover up to about 100,000 individual samples, and particularly about 1,000 to about 100,000, or about 5,000 to about 20,000, e.g., about 10,000 individual samples.

In particular embodiments, a plurality of individual evanescent fields is generated. These individual evanescent fields may or may not overlap. In certain embodiments, the individual evanescent fields substantially do not overlap. In certain embodiments, the overlap between two adjacent evanescent fields is about 20% (by area) or less, or about 10% (by area) or less.

In certain embodiments, the detecting means comprises a detector matrix comprising a plurality of detection pixels. The detection pixels may be optically projected on the sample spots on the surface of the support. In particular embodiments, the detector provides an optical focus on the individual sample spots in order to achieve the desired accuracy in the measurement of single molecule events occurring at the sample spots. The essential part of the innovation is to create many individual evanescent fields of excitation energy from splitting one laser beam by a diffractive optical element.

In certain embodiments, least one reaction space is provided around said plurality of samples, wherein said reaction space comprises a medium, e.g., liquid medium, and particularly an aqueous liquid medium and components for said single molecule event. In particular embodiments, the reaction space is a flow cell thereby providing a flow of fresh components to the sample spots and a flow of spent components from the sample spots. In particular embodiments, a single reaction space is provided around the plurality of samples. In further embodiments, individual reaction spaces are provided around each sample or around groups of samples. For example, a reaction space has a depth of about 10 nm to about 1,000 µm.

In particular embodiments, the emission radiation pathway passes from the sample spot through the reaction space to the detection means.

In further particular embodiments, the radiation source is located above an optically transparent support comprising a plurality of sample spots at its lower surface, i.e., the surface distal to the radiation source. The lower surface of the support comprising the sample spot is in contact with a reaction space, e.g., a single reaction space which may be a flow cell as described above. The radiation source emits excitation radiation downward into the direction of the support. The excitation radiation is split into multiple individual beams which then enter the support at its upper surface, i.e., the surface proximal to the radiation source. The individual radiation beams are reflected at the lower surface and thereby generate individual evanescent fields, e.g., by total internal reflection, at the sample spots or groups of sample spots as described above. The emission radiation pathway from the sample spots is directed downward through the reaction space to the detection means.

In certain embodiments, the method of the present disclosure comprising analyzing multiple subsequent single molecule events in one sample, particularly comprising separately analyzing multiple subsequent single molecule events in different samples in parallel. The term "multiple single molecule events" encompasses a sequence of consecutive single molecule events that take place at the same sample, e.g., at the sample spot on a support. In certain embodiments, the sequence of consecutive single molecule events comprises up to 10, up to 100, up to 1,000, up to 10,000 or even more individual single molecule events, e.g., up to about 10,000,000 or 100,000,000 individual single molecule events. For example, multiple single molecule events may comprise subsequent nucleic acid elongation and/or degradation steps of a single molecule nucleic acid sequence analysis.

In particular embodiments, a single molecule nucleic acid sequence analysis comprises multiple steps of nucleic acid elongation, wherein a luminescent nucleotide building block, e.g., a luminescence-labeled nucleoside polyphosphate, e.g., comprising 3 to 15 phosphate groups is incorporated into a nucleic acid molecule in the presence of a nucleic acid-polymerizing enzyme, e.g., a DNA or RNA polymerase. Those embodiments may comprise detecting emission radiation from the incorporation of a luminescent nucleotide building block, e.g., a luminescence-labeled nucleoside polyphosphate into said nucleic acid molecule.

The present disclosure further provides a device for analyzing a single molecule event comprising:
- means for providing a plurality of samples adapted for a single molecule event,
- an optical excitation device comprising a radiation source adapted for emitting excitation radiation, a diffractive optical element for splitting said excitation radiation into multiple individual radiation beams and an optical arrangement for directing said multiple individual radiation beams to said plurality of samples,
- a detection means adapted for capturing emission radiation from said plurality of samples, and
- an analyzing means adapted for analyzing the captured emission radiation,
wherein the excitation radiation pathway from said radiation source to said plurality of samples and the emission radiation pathway from said plurality of samples to said detection means do not overlap.

In certain embodiments, the device is adapted for performing the method as described above, e.g., for performing a single molecule nucleic acid sequence analysis.

The method and the device of the present disclosure are useful for providing a high-throughput analysis of a single molecule event. In certain embodiments, the high-throughput analysis comprises a parallel analysis of about 10⁶ to about 10¹¹ individual samples, particularly a parallel analysis of about 10⁶ to about 10¹¹ individual sample spots on a single support, e.g., on a planar support. In certain embodiments, the high-throughput analysis is carried out in a single reaction space.

Methods and devices for analyzing single molecule events are e.g., disclosed in WO 2002/097406, WO 2003/052137, WO 2006/013110, WO 2013/131888, WO 2015/104245, WO 2017/001407, and WO 2018/104301, the contents of which are herein incorporated by reference.

For the analysis of a single molecule event, the biomolecules may be located at the sample spots on the support. There they are in contact with a sample liquid, which contains the free reaction partners. Thereby, one or more reaction spaces are defined. Particularly at least 100, at least 1000, or at least 10 000, and up to more than 10⁶ molecules may be analysed on a single support, e.g., a single planar support

A nucleic acid molecule whose sequence is to be determined may be selected, for example, from DNA molecules such as genomic DNA fragments, cDNA molecules, plasmids, etc., or else from RNA molecules such as mRNA molecules. The nucleic acid molecule may originate from genomic or expression libraries, generated from cells or organisms, e.g., eukaryotic or prokaryotic cells or organisms. This allows parallel sequencing of a plurality of different nucleic acid template molecules, e.g., at least 10, 100, 1.000 or 10.000 and up to 100.000, 10⁶ or 10⁷ or even more different nucleic acid molecules.

The nucleic acid molecules to be sequenced may be single-stranded nucleic acid molecules in a linear or circular form, e.g., in a covalently linked circular form. In order to obtain a circular nucleic acid template, a linear nucleic acid molecule may be subjected to a circularization procedure and optionally a strand-separation procedure during sample preparation. Circularization may be effected by ligation according to known protocols, e.g., using DNA or RNA ligases. In some embodiments, an adaptor and/or identifier molecule, i.e., a nucleic acid molecule of known sequence, may be coupled to the nucleic acid molecule.

The sequence determination may comprise nucleic acid elongation and/or nucleic acid degradation. The sequencing process includes one or more sequencing cycles.

The nucleic acid-synthesizing enzyme molecules are capable of elongating a primer annealed to a nucleic acid template molecule. Primer elongation may be carried out by progressively incorporating individual nucleotide building blocks at the 3'-terminus of a growing nucleic acid chain, wherein a nucleic acid molecule complementary to the sequence of the circular nucleic acid template is generated. The nucleic acid-synthesizing enzymes are selected from polymerases capable of a template specific nucleic acid polymerization, preferably from DNA polymerases and RNA polymerases, e.g., natural or modified polymerases, including thermostable DNA polymerases.

Specific examples of suitable DNA polymerases include Taq polymerases, exonuclease-deficient Taq polymerases, E. coli DNA polymerase I, Klenow fragment, reverse transcriptase, Φ29-related polymerases including wild-type Φ29 polymerase and derivatives of such polymerases, such as exonuclease-deficient forms, T7 DNA polymerase, T5 DNA polymerase, an RB69 polymerase and others.

Nucleic acid-degrading enzyme molecules are capable of progressively cleaving off individual nucleotide building blocks from a nucleic acid molecule. Preferably exonucleases, more preferably single-strand exonucleases which degrade in the 3'→5' direction or in the 5'→3' direction are used. Exonucleases which are particularly preferably used are 3'→5' exonucleases such as E. coli exonuclease I and E. coli exonuclease III, and 5'→3' exonucleases such as T7 exonuclease, E. coli exonuclease II and E. coli exonuclease VIII. Further, the exonuclease activities of various polymerases, e.g., the Klenow fragment, Taq polymerase or T4 polymerase may be used.

The nucleic acid-synthesizing enzyme molecules are contacted with a linear or circular nucleic acid template molecule, e.g., a single-stranded DNA or RNA molecule, and a primer molecule annealed to the nucleic acid template molecule or capable of annealing thereto. The primer molecule is preferably a single-stranded nucleic acid or nucleic acid analogue molecule having a free 3'-end which can be extended by an enzymatic reaction catalyzed by the immobilized nucleic acid-synthesizing enzyme molecules. The length of the primer molecule is selected to allow effective annealing to the template under reaction conditions. Usually, the length of the primer molecule is at least 8, at least 10, at least 12 or at least 15 nucleotides and e.g., up to 20, 25, 50 or 100 nucleotides, or even higher. In some embodiments, the primer is resistant against digestion by nucleic acid-degrading enzyme molecules, e.g., by incorporating nucleotide analogue building blocks and/or linkages between nucleotide building blocks, which are stable against degradation. In other embodiments, the primer is sensitive against digestion by nucleic acid-degrading enzyme molecules.

The sequence of the primer is selected in that it effectively anneals under reaction conditions to the template molecule. For instance, the primer may be a universal degenerated primer capable of statistically annealing to unknown nucleic acid sequences. In other embodiments, the primer may be capable of annealing to a known sequence portion of the nucleic acid template molecule. In this embodiment, a known adaptor and/or identifier sequence may be incorporated into the nucleic acid template molecule. The primer may be unlabelled or comprise fluorescent labelling groups.

Further, the presence of luminescent nucleotide building blocks, e.g., nucleotide building blocks carrying at least one fluorescent labelling group is required. Preferably, each different nucleotide building block (A, G, C, T/U) contains a different fluorescent labelling group.

The fluorescent labelling groups may be selected from known fluorescent labelling groups used for labelling biopolymers, particularly nucleic acids, such as, for example, fluorescein dyes, rhodamines, oxazines, for example Evoblue or Gnothis Blue, phycoerythrin, Cy3, Cy5, IR dyes or derivatives thereof, etc.

The nucleotide building blocks may carry (i) a fluorescence labelling group which remains with the building block when the building block is incorporated into a nucleic acid molecule during a primer elongation catalyzed by a nucleic acid-synthesizing enzyme molecule, and/or (ii) a fluorescence labelling group which is cleaved off from the building block when the building block is incorporated into a nucleic acid molecule during a primer elongation catalyzed by a nucleic acid-synthesizing enzyme molecule. Fluorescence labelling groups remaining with the building block are preferably attached to the α-phosphate group, to the sugar and/or to the nucleobase group.

In particular embodiments, fluorescence labelling groups remaining with the building block are attached to the nucleobase, e.g., via a linker which may have a chain-length of up to 15, preferably of 10-12 carbon atoms, optionally including heteroatoms, e.g., N, O or S atoms. Fluorescence labelling groups which are cleaved off when the building block is incorporated into a nucleic acid molecule may be attached to a terminal phosphate group, e.g., of a polyphosphate building block including, but not limited to a hexa-, penta-, tetra- or triphosphate building block such as the γ-phosphate group of a triphosphate building block. In certain embodiments, building blocks are selected which contain both (i) a fluorescence labelling group remaining after incorporation and (ii) a fluorescence labelling group cleaved off during incorporation. In this case, fluorescence groups capable of interacting with each other, e.g., by quenching and/or energy transfer, may be selected.

The nucleic acid molecules to be sequenced will contain fluorescent labelling groups in case the nucleic acid molecule is subjected to direct sequencing using a nucleic acid-degrading enzyme molecule. On the other hand, the nucleic acid molecule to be sequenced my not contain fluorescent labelling groups, if the nucleic acid molecule is used as a template in a primer elongation.

The sequencing procedure may involve a step of generating nucleic acid molecules having incorporated nucleotide building blocks in a primer elongation catalyzed by the nucleic acid-synthesizing enzyme molecules and/or a second step of cleaving off individual nucleotide building blocks from the generated nucleic acid molecules catalyzed by nucleic acid-degrading enzyme molecules. Dependent on the type of fluorescence labels, nucleic acid sequence determination may be carried out during primer elongation and/or during degradation.

Sequence determination during the primer elongation involves the use of nucleotide building blocks carrying a fluorescence-labelling group which is cleaved off from the building block when it is incorporated into a nucleic acid molecule. In this case, a time-dependent fluorescence change caused by cleaving off the fluorescence-labelling group from the nucleotide building block may be determined. Sequence determination during nucleic acid degradation involves the use of a nucleotide building block, which carries a fluorescence-labelling group which remains with the building block when it is incorporated into a nucleic acid molecule. Progressive cleavage of individual nucleotide building blocks from the nucleic acid molecules causes a time-dependent change of fluorescence when the labelled nucleotide building block is liberated from the nucleic acid molecule. In certain embodiments, it is also possible to carry out a sequence determination during elongation and degradation, i.e., when using nucleotide building blocks, which both carry a fluorescence-labelling group remaining with the building block and a fluorescence-labelling group which is cleaved off from the building block when the building block is incorporated into a nucleic acid molecule. In this embodiment, both fluorescent groups may be the same or different.

In some embodiments, the method involves one or more cycles of nucleic acid-synthesis and nucleic acid-degradation in order to determine the base sequence of a nucleic acid molecule template. The nucleic acid synthesis involves an elongation of the primer annealed to the nucleic acid template molecule catalyzed by the nucleic acid-synthesizing enzyme molecule, wherein a nucleic acid molecule complementary to the sequence of the nucleic acid template is generated. In the next step, the generated nucleic acid molecule is degraded by a nucleic acid-degrading enzyme molecule.

When a nucleotide building block is incorporated into an elongated nucleic acid molecule, a time dependent change in the fluorescence may occur, which can be detected as indicated above. Preferably, the incorporation of the nucleotide building blocks into the elongated nucleic acid molecule is associated with a detectable increase in the fluorescence, preferably with a transient increase in the fluorescence. For example, nucleotide building blocks may be used which carry a fluorescent labelling group on the portion of the molecule which is cleaved off when the building block is incorporated into the primer, e.g., on the γ-phosphate group.

When a nucleotide building block is cleaved off from the synthesized nucleic acid molecule, a time-dependent change of fluorescence may be determined due to the interaction of fluorescent labelling groups incorporated in nucleic acid strands with neighbouring groups, for example with chemical groups of the nucleic acids, in particular nucleobases such as, for example, G, or/and neighbouring fluorescent labelling groups, and these interactions leading to a change in fluorescence, in particular in fluorescence intensity, compared to the fluorescent labelling groups in "isolated" form, owing to quenching processes or/and energy transfer processes. The removal by cleavage of individual nucleotide building blocks alters the overall fluorescence, for example the fluorescence intensity of an immobilized nucleic acid strand, and this change is a function of the removal by cleavage of individual nucleotide building blocks, i.e., a function of time.

In certain embodiments, association of a labelled nucleotide with the biomolecule complex is detected by measuring polarisation of the emitted photons. The polarisation of excited states' photons is changed by the rotational movement of the light emitting nucleotide labels and can be used for identifying free moving contra bound labelled nucleotides in the polymerisation process.

This time-dependent change in fluorescence during elongation and/or degradation may be recorded in parallel for a multiplicity of nucleic acid molecules and correlated with the base sequence of the individual nucleic acid strands. Preference is given to using those fluorescent labelling groups which, when incorporated in the nucleic acid strand, are, at least partially, quenched so that the fluorescence intensity is increased after the nucleotide building block containing the labelling group or a neighbouring building block causing quenching has been removed by cleavage.

During incorporation and/or removal of individual nucleotide building blocks, it is possible to measure a change in fluorescence intensity of the nucleic acid strand or/and the incorporated or cleaved-off nucleotide building block, owing to quenching processes or energy transfer processes. This change in fluorescence intensity with time depends on the base sequence of the nucleic acid strand studied and can therefore be correlated with the sequence.

The complete sequence of the nucleic acid molecule may be determined by using a mixture of nucleotide building blocks, labelled on all four different bases, for example on A, G, C and T, or on combinations of two or three different bases. It is possible, where appropriate, to attach to the nucleic acid strand to be studied also a "sequence identifier", i.e., a labelled nucleic acid of known sequence, for example by enzymatic reaction using ligase or/and terminal transferase, so that at the start of sequencing initially a known fluorescence pattern and only thereafter the fluorescence pattern corresponding to the unknown sequence to be studied is obtained.

The detection comprises irradiating excitation radiation from a radiation source into the support, preferably by means of a laser, or by another suitable light source, in order to cause excitation of the fluorescent labelling groups. In certain embodiments, the radiation source comprises a plurality of different lasers emitting radiation at different wavelengths. It is possible, in this connection, to use one or more laser beams, for example an expanded laser beam, having a cross section of approx. 1-20 mm, and/or multiple laser beams. The detection preferably comprises a multipoint fluorescence excitation by lasers, for example a dot matrix of laser dots generated via diffraction optics (cf. WO 2002/097406) or a quantum well laser.

Fluorescence emission of a plurality of nucleic acid strands may be detected in parallel using a detecting means, e.g., a detector matrix which comprises, for example, an electronic detector matrix, for example a CCD camera, a CMOS detector matrix, e.g., a CMOS camera, or an avalanche photodiode matrix. The detection may be carried out in such a way that fluorescence excitation and detection are carried out in parallel on a part or all nucleic acid strands studied. Preference is given to carrying out the detection on fluorescence light which is emitted essentially orthogonally from the support surface through the reaction space or through the support body.

The detection may be carried out, for example, by means of a single molecule detection, for example by fluorescence correlation spectroscopy, which involves exposing a very small, preferably confocal, volume element, for example from 10⁻²¹ to 10⁻¹⁰ I, to the excitation light of a laser, or another suitable light source, which light excites the receptors present in this measuring volume so that the latter emit fluorescence light, the fluorescence light emitted from said measuring volume being measured by means of a photodetector and the change in the measured emission with time being correlated with the concentration of the analyte, so that it is possible to identify, at an appropriately high dilution, individual molecules in said measuring volume. Details of the procedure and of the apparatus used for detection can be found in the disclosure of EP 0 679 251, the content of which is herein incorporated by reference. The confocal determination of single molecules is furthermore described in Rigler and Mets (Soc. Photo-Opt. Instrum. Eng. 1921 (1993), 239 ff.) and Mets and Rigler (J. Fluoresc. 4 (1994) 259-264), the contents of which are herein incorporated by reference.

Alternatively, or additionally, detection may also be carried out by way of time-resolved decay measurement, called "time gating", as described, for example, by Rigler et al., "Picosecond Single Photon Fluorescence Spectroscopy of Nucleic Acids", in: "Ultrafast Phenomena", D.H. Auston, Ed., Springer 1984, the content of which is herein incorporated by reference. Here, the fluorescent molecules are excited in a measuring volume followed by, e.g., at a time interval of ≥ 100 ps, opening a detection interval on the photodetector. In this way it is possible to keep background signals generated by Raman effects sufficiently low so as to enable single molecules to be detected in an essentially interference-free manner.

Further, the present disclosure is explained in detail by reference to the following specific embodiments.

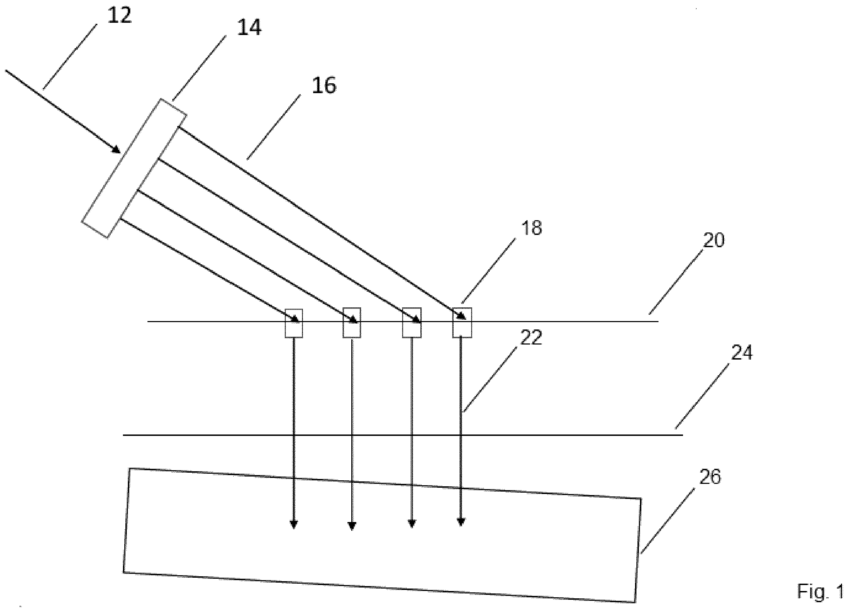

Fig. 1 shows an embodiment of the present disclosure. A laser beam (12) is split into a plurality of individual beams, e.g., four individual beams (16), by means of a diffractive optical element (14). The individual beams (14) are directed to sample spots (18) located on a support (20) where the individual beams (14) produce a predetermined pattern of optical foci. Light (22) emitted from the foci at the sample spots (18) is directed by an optical arrangement (24) to a detector (26). The difference between this embodiment and prior art embodiment (previous slide) is that the radiation source light pathway and the emission light pathway are not the same during throughout the pathway and do not overlap.

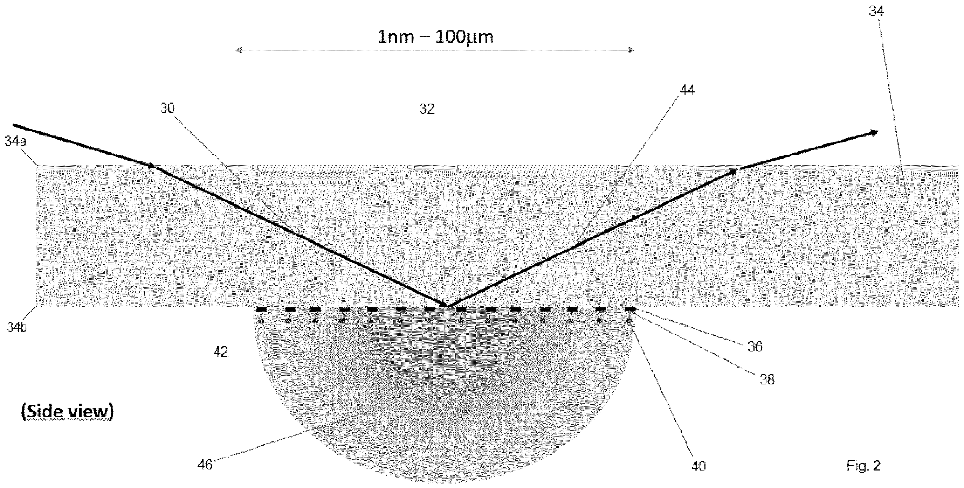

Fig. 2 shows a further embodiment of the present disclosure where the detection is based on an evanescent field. In Fig. 2, a single evanescent field, e.g., 1 out of 10,000 evanescent fields in total is depicted.

An incoming individual light beam (30) generated by passing excitation light through an optical diffractive element (not shown) is passed through a gaseous medium (32) to an optically transparent support (34), e.g., made of silicon dioxide. It enters the support (34) at a surface (34a) that is distal to the surface (34b) where sample spots (36) are located. To the surface of the sample spots (36) binding molecules (38) are attached that mediate immobilization of a single biomolecule or single molecule complex (40), e.g., a DNA or RNA polymerase, or a complex comprising a DNA or RNA polymerase and a nucleic acid template and primer. The sample spots (36) are surrounded by a reaction space (42) comprising a medium, e.g., an aqueous medium and components that are required for the single molecule event, e.g., fluorescence labelled nucleotide building blocks. The incoming light beam (30) is reflected at the surface (34b). Thereby, totally reflected light (44) and an evanescent field (46) is generated. The evanescent field (46) encompasses a single sample spot (36) or - as shown here - a group of sample spots (36), e.g., up to 100,000 and typically about 10,000 sample spots, and may have a diameter of e.g., 1 nm to 100 µm. Since the excitation light is split into multiple individual beams, multiple evanescent fields may be generated at the support surface (34b). Emission light from the single molecule events occurring on sample spots (36) is passed to a detector (not shown).

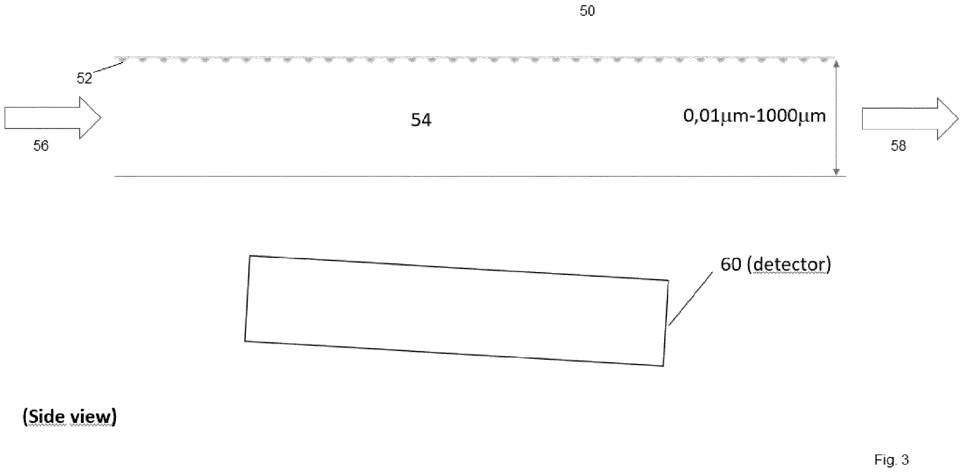

Fig. 3 shows a further embodiment of the present disclosure. A support (50) is provided that has sample spots (52) with single molecules immobilized thereon (not shown) on its surface. The sample spots (52) are in contact with a single reaction space (54) that is a flow cell comprising an inlet (56) for the inflow of medium comprising fresh components for interacting with the single molecules and an outlet (58) for the outflow of medium comprising spent components. Emission light (not shown) from the sample spots (52) passes through the reaction space (54) to a detector (60). The detector comprises pixels that are optically projected on the sample spots (52) on the surface of the support (50).

## Claims

1. A method for analyzing a single molecule event comprising:
(a) providing a plurality of samples where a single molecule event takes place,
(b) irradiating said plurality of samples with an optical excitation device comprising a radiation source, a diffractive optical element for splitting excitation radiation into multiple individual radiation beams and an optical arrangement for directing said multiple individual radiation beams to said plurality of samples,
(c) capturing emission radiation from said plurality of samples by a detection means, and
(d) analyzing the captured emission radiation,
wherein the excitation radiation pathway from said radiation source to said plurality of samples and the emission radiation pathway from said plurality of samples to said detection means do not overlap.

2. The method of claim 1, wherein the excitation radiation pathway from said radiation source originates from a plane above the emission pathway from said plurality of samples to said detection means.

3. The method of claim 1 or 2, wherein said plurality of samples is present on a support, wherein said support comprises an optically transparent substrate and a plurality of sample spots on the surface of the support, wherein the sample spots are spatially separated from another and wherein said samples are present on said sample spots.

4. The method of claim 3, wherein a component of said single molecule event is immobilized to said at least one sample spot, and wherein said immobilized component comprises a single biomolecule.

5. The method of any one of the preceding claims, wherein an individual radiation beam of the excitation radiation is directed to an individual sample or to a group comprising multiple individual samples, wherein said group comprises up to about 10⁶ individual samples, and particularly about 1,000 to about 10⁶, or about 5,000 to about 10⁵, e.g., about 10,000 individual samples.

6. The method of any one of the preceding claims, wherein said multiple beams generated by the optical diffractive element are passed to said plurality of samples through an optically transparent substrate of a support on which the plurality of samples is located, wherein the plurality of samples is located on the surface of the support that is distal to the surface of the support through which the excitation radiation enters the support.

7. The method of claim 6, wherein an individual radiation beam of the excitation radiation generates, e.g. by total internal reflection, an individual evanescent field on the surface of the support on which the plurality of samples is located, wherein said individual evanescent field has a diameter of about 1 nm to about 100 µm or about 10 nm to about 10 µm and wherein said individual evanescent field covers up to about 10⁶ individual samples, and particularly about 1,000 to about 10⁶, or about 5,000 to about 10⁵, e.g., about 10,000 individual samples.

8. The method of any one of the preceding claims, wherein said detection means comprises a detector matrix comprising a plurality of detection pixels, wherein said detection pixels are optically projected on the sample spots on the surface of the support.

9. The method of any one of the preceding claims, wherein at least one reaction space is provided around said plurality of samples, wherein said reaction space comprises a medium and components for said single molecule event.

10. The method of claim 9, wherein said emission radiation pathway passes through said reaction space.

11. The method of any one of the preceding claims, wherein said single molecule event comprises a sequence analysis of a single nucleic acid molecule.

12. A device for analyzing a single molecule event comprising:
- means for providing a plurality of samples adapted for a single molecule event,
- an optical excitation device comprising a radiation source adapted for emitting excitation radiation, a diffractive optical element for splitting said excitation radiation into multiple individual radiation beams and an optical arrangement for directing said multiple individual radiation beams to said plurality of samples,
- a detection means adapted for capturing emission radiation from said plurality of samples, and
- an analyzing means adapted for analyzing the captured emission radiation, wherein the excitation radiation pathway from said radiation source to said plurality of samples and the emission radiation pathway from said plurality of samples to said detection means do not overlap.

13. The device of claim 12 adapted for performing a single molecule nucleic acid sequence analysis.

14. Use of the method of any one of claims 1-11 or the device of any one of claims 12-13 for providing a high-throughput analysis of a single molecule event.

15. The use of claim 14, wherein the high-throughput analysis comprises a parallel analysis of about 10⁶ to about 10¹¹ individual sample spots on a single support.
